(19) Europäisches Patentamt / European Patent Office / Office européen des brevets

(11) **EP 1 904 533 B1**

(12) **EUROPÄISCHE PATENTSCHRIFT**

(45) Veröffentlichungstag und Bekanntmachung des Hinweises auf die Patenterteilung:
**04.03.2015 Patentblatt 2015/10**

(21) Anmeldenummer: **06743127.0**

(22) Anmeldetag: **08.06.2006**

(51) Int Cl.:
*A61Q 5/12* (2006.01)    *A61K 8/73* (2006.01)
*C08B 37/00* (2006.01)

(86) Internationale Anmeldenummer:
**PCT/EP2006/005461**

(87) Internationale Veröffentlichungsnummer:
**WO 2006/133845 (21.12.2006 Gazette 2006/51)**

(54) **AMINOALKYLGRUPPENHALTIGE GUAR-DERIVATE**

GUAR GUM DERIVATIVES CONTAINING AMINO ALKYL GROUPS

DERIVES DE GUAR CONTENANT DES GROUPES AMINOALKYLE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **15.06.2005 DE 102005027498**

(43) Veröffentlichungstag der Anmeldung:
**02.04.2008 Patentblatt 2008/14**

(73) Patentinhaber: **Dow Global Technologies LLC**
**Midland, MI 48674 (US)**

(72) Erfinder:
• **HÜTTERMANN, Carsten**
  **38102 Braunschweig (DE)**
• **BRACKHAGEN, Meinolf**
  **29665 Walsrode (DE)**
• **ENGELHARDT, Jürgen**
  **29683 Bad Fallingbostel (DE)**

(74) Vertreter: **f & e patent**
**Fleischer, Engels & Partner mbB, Patentanwälte**
**Braunsberger Feld 29**
**51429 Bergisch Gladbach (DE)**

(56) Entgegenhaltungen:
**WO-A-2006/010470       US-A- 5 739 304**
**US-A1- 2001 051 143**

**Beschreibung**

Die Erfindung betrifft aminoalkylgruppenhaltige Guar-Derivate und ein Verfahren zur Herstellung dieser Polysaccharidderivate durch Veretherung freier Hydroxylgruppen von Guar oder Guar-Derivaten mit Dialkylaminoalkylhalogeniden.

Kationische und aminogruppenhaltige Polysaccharide können vielfältig eingesetzt werden, z.B. als Papierhilfsmittel, als Chelatisierungs- und Flockungsmittel oder als Konditionierer für Haare. Insbesondere Chitosan, 2-Amino-2-desoxy-cellulose, findet breite Verwendungsmöglichkeiten, unter anderem als säurestabiler Verdicker in kosmetischen Zubereitungen. Die Gewinnung dieses natürlichen Polysaccharides ist jedoch ein aufwändiger Prozess, was sich in hohen Preisen der Produkte widerspiegelt. Der hohe Preis steht bisher einer breiten Anwendung dieser Polysaccharide und Polysaccharidderivate entgegen.

Aus dem Stand der Technik sind bereits einige Möglichkeiten zur Herstellung aminoalkylgruppenhaltiger Guar-Derivate bekannt.

In EP 0 175 113 A2 werden Polysaccharidderivate beschrieben, die durch Umsetzung von Polysacchariden oder Polysaccharidderivaten mit acetalgruppenhaltigen Substanzen hergestellt werden. In einem Beispiel wird als Polysaccharidderivat-Basis Diethylaminoethylguar mit einem niedrigen Substitutionsgrad (DS 0,12) eingesetzt. Es werden keine Angaben zu den Eigenschaften des Diethylaminoethylguar gemacht.

In DE 28 40 011 wird ein Verfahren zur Herstellung von Diethylaminoethylguar beschrieben, das dadurch gekennzeichnet ist, dass die wässrige Reaktionsphase in einem mit Wasser nicht mischbaren Lösungsmittel unter Zusatz eines Tensids emulgiert wird. Das Verfahren hat den Nachteil, dass erhebliche Mengen Tensid verwendet werden. Die durch das Verfahren beanspruchten Produkte haben den Nachteil, dass sie ein niedrigeres Molekulargewicht und eine wesentlich geringere Viskosität aufweisen als die erfindungsmäßigen Produkte.

In US 4,276,414 wird in Beispiel 4 die Umsetzung von Guar mit 0,04 mol Diethylaminoethylchlorid pro mol Anhydrozucker in wässrigem Isopropanol beschrieben. Es werden jedoch keine Angaben zu den Eigenschaften dieses gering substituierten Diethylaminoethylguar erwähnt.

In US 3,498,912 wird die Herstellung von Alkylaminoalkylguar zur Behandlung von Abwasser beschrieben. Bevorzugt sind dabei die Umsetzungen von Guar mit Aminen, die Epoxy- oder Halohydrin ($XCH_2$-CH(OH)-R) -Gruppen enthalten. Sie weisen einen Substitutionsgrad DS(N) von 0,01 bis 0,5 auf. Höhere Substitutionsgrade sind schwieriger zu erreichen und nicht bevorzugt. Die Herstellung des Alkylaminoalkylguar erfolgt in einem alkoholischen Lösungsmittel.

US 2001/0051143 A1 offenbart kationisch modifizierten Guar mit einem DS von 0,25 bis 1,0, der erhalten wird durch Reaktion in einem alkoholischen Medium.

Die bisher bekannten Verfahren und ihre Produkte weisen jedoch einige Mängel auf. So fuhren die bekannten Verfahren insbesondere zu Produkten, die nicht ausreichend klarlöslich sind und die - bei gegebener Ausgangsviskosität - eine für viele Anwendungen zu geringe Endviskosität (und entsprechend geringes Molekulargewicht) aufweisen. Ein hohes Molekulargewicht ist für viele Anwendungen, in denen kationische Polysaccharide eingesetzt werden können, von Bedeutung. Beispiele dafür sind die Abwasserreinigung oder Haarkonditionierung.

Aufgabe der Erfindung war es daher, kostengünstige, hochviskose aminogruppenhaltige, hochmolekulare Polysaccharidderivate zu entwickeln, die bereits bei geringem Substitutionsgrad klar löslich sind.

Es wurde nun gefunden, dass durch die nachfolgend beschriebene Veretherung von Guar oder Guar-Derivaten mit Aminoalkylchloriden, z.B. Diethylaminoethylchlorid-Hydrochlorid (DEC), in einem Slurry-Verfahren Dialkylaminoalkylguar mit überraschend hoher Ausbeute hergestellt werden kann.

Die Produkte können mit wenig Aufwand gereinigt werden und weisen folgende Vorteile gegenüber dem Stand der Technik auf:

1) Diese aminogruppenhaltigen Polysaccharidderivate lösen sich ausgezeichnet in kaltem Wasser mit einer hohen Klarlöslichkeit.

2) Die Produkte haben ein hohes Molekulargewicht.

3) Die wässrigen Lösungen weisen eine hohe Viskosität auf.

Gegenstand der Erfindung ist ein Verfahren zur Herstellung aminoalkylgruppenhaltiger Guar-Derivate, dadurch gekennzeichnet, dass man

a) Guar oder ein Guar-Derivat in einem Gemisch (Slurry) aus einem wassermischbaren aprotischen Lösungsmittel und Wasser dispergiert und

b) das Guar oder Guar-Derivat mit einer Base alkalisiert und

c) anschließend eine Veretherung mit einem aminogruppenhaltigen Reagenz bei gegebenenfalls erhöhter Temperatur durchführt und dann

d) gegebenenfalls neutralisiert und

e) das Reaktionsprodukt gegebenenfalls abfiltriert, gegebenenfalls wäscht, gegebenenfalls trocknet und gegebenenfalls mahlt.

Guar ist ein Mehl, das durch Mahlen des Endosperms der Samen der in Indien beheimateten Pflanze Cyamopsis tetragonolobus gewonnen wird. Hauptbestandteil des Guars ist Guaran, ein nichtionogenes Polysaccharid aus β-(1→4)-glykosidisch verknüpften D-Mannopyranose-Einheiten mit α-(1→6)-verknüpfter D-Galactopyranose in der Seitenkette, und zwar je eine D-Galactose-Einheit auf 2 Mannose-Einheiten.

Anstelle von Guar können auch Guar-Derivate, insbesondere Guarether eingesetzt werden. Beispiele für Guarether sind Hydroxypropylguar, Hydroxyethylguar, Methylguar, Carboxymethylguar oder Guarether mit gemischten Substituenten wie zum Beispiel Hydroxypropylmethylguar oder Hydroxyethylmethylguar.

Für das Slurrymedium (Slurry = Lösungsmittel + Wasser) werden solche Lösungsmittel verwendet, die wassermischbar und aprotisch sind. Dabei handelt es sich bevorzugt um Ketone oder Ether. Besonders bevorzugte Lösungsmittel sind Aceton, Methylethylketon, Dimethoxyethan. Ganz besonders bevorzugt ist Dimethoxyethan.

Der Anteil an Lösungsmittel im Reaktionsmedium sollte hoch genug sein, damit das wasserlösliche Guar oder Guar-Derivat nicht gelöst sondern dispergiert wird. Ebenso sollte der Anteil an Wasser im Reaktionsmedium so hoch sein, dass die Base bei der Alkalisierung im Reaktionsmedium zumindest teilweise gelöst wird. Ein Lösungsmittelanteil im Reaktionsmedium von 40 - 90 % ist bevorzugt.

Die Reaktion wird in Gegenwart eines wassermischbaren aprotischen Lösungsmittels in einem Rührgefäß oder Reaktionsmischer durchgeführt. Die Reaktion kann in einem Rührgefäß durchgeführt werden, wenn das Verhältnis von Polysaccharid zu Slurry bis ca. 1:5, bevorzugt etwa 1:15 bis 1:6 Gewichtsteile beträgt. Bei höheren Verhältnissen von bis zu etwa 1:4 Gewichtsteile wird der Ansatz gegebenenfalls schwerer rührbar. In diesem Fall kann ein Reaktionsmischer eingesetzt werden. Diese werden z.B. unter der Bezeichnung All In One Reactor® (Gebr. Lödige Maschinenbau, Division Drais, Mannheim) oder unter dem Namen DRUVATHERM® Reaktor (Gebr. Lödige Maschinenbau, Paderborn) angeboten. Diese Mischer bestehen im Allgemeinen aus einer horizontal angeordneten, zumeist zylinderförmigen Mischkammer. Darin befindet sich eine Welle, die mit z.B. paddel- oder pflugscharförmigen Mischelementen versehen ist. Dreht sich die Welle, so werden Partikel aus dem Mischgutbett herausgeschleudert und im Raum über dem Mischgut verwirbelt und vermischt. Der Mischer kann gegebenenfalls mit weiteren Einbauten, wie z.B. sogenannte Messerköpfen oder Düsen versehen sein. [Karl Sommer in: "Mixing of Solids, 3.

Designs of Solid - Solid Mixers, 3.3 Paddle Mixers", Ullmann's Encyclopedia of Industrial Chemistry, Wiley-VCH Verlag GmbH & Co. KgaA, 2002. DOI: 10.1002/14356007.b02_27, Article Online Posting Date: June 15, 2000]

Das Guar oder Guar-Derivat wird vor der Zugabe des aminogruppenhaltigen Reagenz mit einer Base, bevorzugt ein Alkali- oder Erdalkalimetallhydroxid, besonders bevorzugt Natriumhydroxid, vermischt. Die Reihenfolge der Zugabe kann aber auch umgekehrt sein. Die Base kann dabei in fester Form oder in Form einer Lösung zugegeben werden. Werden ausschließlich feste Basen verwendet, so muss ggf. etwas Wasser hinzugefügt werden, da eine bestimmte Menge an Wasser während der Alkalisierung im System vorhanden sein muss.

Wenn das freie Amin eingesetzt wird, kann während der Veretherung entstehende Säure durch die Aminogruppe gebunden werden. Es sollten jedoch bevorzugt mindestens 0,5 Äquivalente Base pro mol Amin eingesetzt werden, besonders bevorzugt mindestens 0,7 Äquivalente, um eine ausreichende Reaktionsgeschwindigkeit zu erzielen.

Höchstens sollten 1,5 Äquivalente Base pro mol Amin eingesetzt werden, bevorzugt höchstens 1,2 mol und in einer besonders bevorzugten Ausführungsform 0,8 - 1,1 mol.

Die Basenmenge muss entsprechend erhöht werden, wenn das aminogruppenhaltige Reagenz in Form eines Ammoniumsalzes z.B. als Hydrochlorid eingesetzt wird. Dann muss zusätzlich mindestens eine äquimolare Menge, bezogen auf das Ammoniumsalz, an Base hinzugefügt werden, um das Amin, z.B. aus dem Hydrochlorid, freizusetzen.

Als aminogruppenhaltiges Reagenz eignen sich Verbindungen der allgemeinen Formel

$$X\text{-}(CH_2)_n\text{-}NR_1R_2$$

wobei

X    Fluchtgruppe, bevorzugt Chlor, Brom, Iod oder ein Sulfonsäurerest $R'SO_3$, wobei R' ein aromatischer oder aliphatischer Rest ist, z.B. para-Toluyl oder Methyl,

X    vorzugsweise aber Chlor ist und

n   eine gauze Zahl ist, die mindestens 2 sein muss,

die Reste $R_1$ und $R_2$ unabhängig voneinander aliphatische oder verzweigte oder zyklische, gegebenenfalls mit Heteroatomen substituierte Alkyl- oder Aryl-Substituenten mit 1-24 C-Atomen oder H bedeuten. Zwei Reste $R_1$ und $R_2$ können gemeinsam mit dem Stickstoff einen Ring bilden.

Beispiele für erfindungsgemäß zu verwendende aminogruppenhaltige Reagenzien sind N-(2-Chlorethyl)-N,N-diisopropylamin,   N-(2-Chlorethyl)-N,N-diethylamin,   N-(3-Chlorpropyl)-N,N-dimethylamin,   N-(3-Chlorpropyl)-N,N-diethylamin, N-(2-Chlorethyl)-N,N-dimethylamin und N-(2-Chlorpropyl)-N,N-dimethylamin. Die Reste $R_1$ und $R_2$ können zusammen mit dem Stickstoff einen zyklischen Rest bilden. Beispiele für erfindungsgemäß eingesetzte aminogruppenhaltige Reagenzien, die in denen zwei Reste $R_1$ und $R_2$ gemeinsam mit dem Stickstoff einen Ring bilden, sind N-(2-Chlorethyl)-pyrrolidin, N-(2-Chlorethyl)-piperidin und N-(2-Chlorethyl)-morpholin. Die aminogruppenhaltigen Reagenzien können in Form der Ammoniumsalze, z.B. eines Hydrochlorids eingesetzt werden. Sowohl der Feststoff als auch eine Lösung von z.B. 65 Gew.-% oder 50 Gew.-% in Wasser oder einem anderen Lösungsmittel können verwendet werden.

Bevorzugt werden N-(2-Chlorethyl)-N,N-diisopropylamin, N-(2-Chlorethyl)-N,N-diethylamin Hydrochlorid oder N-(2-Chlorethyl)-N,N-dimethylamin Hydrochlorid eingesetzt.

Pro mol Anhydrozucker werden 0,1 - 3 mol, bevorzugt 0,1 - 2 mol, besonders bevorzugt 0,3 - 1,5 mol aminogruppenhaltiges Reagenz eingesetzt.

Die Reaktion wird bevorzugt bei Temperaturen von 40°C bis etwa 90°C, besonders bevorzugt 60°C bis etwa 75°C durchgeführt. Die Reaktionsdauer hängt von dem Reaktor und der eingesetzten Menge an Aminoalkylderivat ab und beträgt bevorzugt von 30 Minuten bis 4 Stunden, besonders bevorzugt 2 bis 4 Stunden.

Nach der Reaktion wird ggf. das Produkt z.B. durch Filtration von Salzen und Nebenprodukten befreit. Dazu werden ggf. vor oder nach der Filtration Säuren wie Ameisensäure, Essigsäure, Salzsäure oder Schwefelsäure zugesetzt. Der Filterkuchen wird ggf. mit einem geeigneten Waschmedium gewaschen. Als bevorzugte Waschmedien haben sich neben den wassermischbaren aprotischen Lösungsmitteln auch Alkohole, wie zum Beispiel Isopropanol, Ethanol oder Methanol, oder Ketone, wie zum Beispiel Aceton, und deren Gemische mit Wasser bewährt. Ein bevorzugtes Waschmedium ist ein Isopropanol - Wasser - Gemisch im Verhältnis Isopropanol : Wasser 3:2 bis 9:1. Es ist aber auch möglich, die Nebenprodukte nicht zu entfernen und das Produkt in ungereinigter Form für technische Anwendungen einzusetzen.

Das aus dem Reaktionsmedium isolierte und ggf. gereinigte Produkt wird ggf. getrocknet und ggf. gemahlen. Dazu können handelsübliche Vorrichtungen verwendet werden.

Die Ausbeute errechnet sich dabei gemäß der in EP-A 0 310 787 erwähnten Formel :

$$\text{Ausbeute (in \%)} = DS_{theor.} / DS_{gem.}$$

$DS_{theor.}$ =   theoretisch möglicher Substitutionsgrad DS, errechnet sich aus dem molaren Verhältnis von aminogruppenhaltigem Reagenz zu Anhydrozucker

$DS_{gem.}$ =   gemessener DS, errechnet sich aus der Formel

$$DS_{gem.} = \frac{\%N \bullet M_{PS}}{(14 \bullet 100) - (\%N \bullet (M_{Ve} - 1))}$$

mit

$M_{PS}$=   Molekulargewichtsanteil einer Monomereinheit des Guars in g/mol

$\%N$ =   gemessener Stickstoffwert in Gew.-%, bezogen auf trockenes Produkt

$M_{Ve}$ =   Molekulargewicht des aminogruppenhaltigen Reagenz in g/mol

Diese Formel liefert jedoch nur exakte Werte, wenn der Stickstoffgehalt sich nur auf das aminogruppenhaltige Polysaccharid bezieht. Die nach der Stickstoffbestimmung (nach Kjeldahl) angegebenen Werte beziehen sich jedoch auf die Gesamtmasse, gegebenenfalls korrigiert um die Feuchtigkeit. Ein genauerer $DS_{gem.}$ errechnet sich, wenn der Stickstoffwert auf Aktivgehalt bezogen wird, d.h. der gemessene Stickstoffgehalt wird um die noch enthaltene Säuren und Salze korrigiert:

$$DS_{gem.} = \frac{\%N \bullet M_{PS} \bullet \frac{Ag}{100}}{(14 \bullet 100) - (\%N \bullet \frac{Ag}{100} \bullet (M_{vc} - 1))}$$

mit Ag = Aktivgehalt, bezogen auf die gesamte Trockenmasse in %

Mit dem erfindungsgemäßen Verfahren können aminogruppenhaltige Guar-Derivate in einfachen, weit verbreiteten Anlagen und in hoher Ausbeute hergestellt werden. Ebenso können die Umsetzungen mit einer hohen Stoffdichte durchgeführt werden. Auf die Zugabe von Tensiden, die anschließend aus dem Produkt entfernt werden müssen, wird verzichtet. Ein weiterer Vorteil ist die einfache Reinigung der Guar-Derivate. Die Produkte lassen sich leicht vom Slurrymedium durch Filtration abtrennen und heterogen reinigen.

Bei den Produkten des erfindungsgemäßen Verfahrens, die einen weiteren Gegenstand der vorliegenden Erfindung bilden, handelt es sich um Guar-Derivate, bei denen ein Teil der Hydroxylgruppen in Dialkylaminoalkylethergruppen der allgemeinen Formel

$$O\text{-}(CH_2)_n\text{-}NR_1R_2$$

umgesetzt wurden, wobei

n    eine gauze Zahl ist, die mindestens 2 sein muss,

die Reste $R_1$ und $R_2$ unabhängig voneinander aliphatische oder verzweigte oder zyklische, gegebenenfalls mit Heteroatomen substituierte Alkyl- oder Aryl-Substituenten oder H bedeuten. Zwei Reste $R_1$ und $R_2$ können gemeinsam mit dem Stickstoff einen Ring bilden.

Der Stickstoff des Substituenten kann, z.B. durch eine weitere Umsetzung mit dem aminogruppenhaltigen Reagenz, auch in quatemisierter Form vorliegen.

Die erfindungsgemäßen Guar-Derivate haben - bei gleichem Molekulargewicht des Ausgangsmaterials - höhere Molekulargewichte als jene, die nach dem Stand der Technik hergestellt wurden. Dieses lässt sich anhand der Staudinger Indices der erfindungsgemäß hergestellten Guar-Derivate im Vergleich zu solchen Produkten, die nach dem Emulsionsverfahren, das in der DE 2 840 011 beschrieben wird, hergestellt wurden, ablesen.

Ein hohes Molekulargewicht ist für viele Anwendungen, in denen kationische Polysaccharide eingesetzt werden können, von Bedeutung. Beispiele dafür sind die Abwasserreinigung oder Haarkonditionierung.

Die erfindungsgemäß hergestellten Dialkylaminoalkylguar-Derivate weisen weiterhin im Vergleich zu Guar-Derivaten, die nach dem Stand der Technik hergestellt wurden, eine hohe Viskosität bei einer hohen Klarlöslichkeit auf.

Die erfindungsgemäß hergestellten Produkte können zum Beispiel als Haarkonditionierer in Shampoos oder als Additiv in kosmetischen Produkten wie zum Beispiel Seifen, Cremes, Lotionen oder Haut- oder Gesichtsreiniger eingesetzt werden. Ebenso können sie als Flockungshilfsmittel zum Beispiel in der Abwasserreinigung verwendet werden. Diese Anwendungen der erfindungsgemäßen Dialkylaminoalkylguar-Derivate bilden einen weiteren Gegenstand der vorliegenden Erfindung.

Die Erfindung soll durch die folgenden Beispiele näher erläutert werden, ohne sie jedoch auf diese Beispiele zu beschränken.

Beispiele

Der angegebene DS bezieht sich auf reinen Dialkylaminoethylguar, d.h. die Gesamtmenge wurde um den Wassergehalt, den Chlorid-Ionengehalt sowie den Natriumionengehalt korrigiert. Der Natriumionengehalt wurde aus der Sulfatasche bestimmt unter der Annahme, dass diese zu 100 % aus $Na_2SO_4$ besteht.

Die Staudinger-Indices wurden durch Viskositätsmessungen in einem Kapillarviskosimeter nach Ubbelohde bei 20°C in 0,1 M NaCl-Lösung ermittelt. Die Auswertung erfolgte nach der Methode nach Huggins.

Die Ladungsdichte bezieht sich auf die Zahl quaternisierter Aminogruppen und wurde durch Polyelektrolyttitration gegen Polyethensulfonsäure Natriumsalz bei pH 11,5 ermittelt.

Die Chemikalien wurden aus dem Handel bezogen. Der Stickstoffgehalt wurde nach der Kjeldahl-Methode bestimmt und basiert auf einer Doppelbestimmung der Probe. Die Viskositäten wurden in einer Lösung von 2 Gew.-% des Polymeren bei einem Schergefälle von 2,55 s$^{-1}$ mit einem Rheometer vom Typ Rotovisko VT 550, Fa.Haake, bestimmt.

**Beispiele 1-6: Herstellung von Diethylaminoethylguar in einem Wasser-Dimethoxyethan-Slurry**

Guar (53 g, Trockengehalt 92,18 %, Staudinger Index: 1392 mL/g) wird in einer Mischung aus Dimethoxyethan (647,8 g) und Wasser (96,3 g) in einer 1 L Glas-Vierhalsplanschliff-Rundboden Rührapparatur mit Impeller-Rührer suspendiert.

Die angegebene Menge Natriumhydroxid wird als Feststoff zugegeben und nach 1 h bei Raumtemperatur wird die angegebene Menge N-(2-Chlorethyl)-N,N-diethylamin Hydrochlorid als wässrige Lösung (65 wt-%) zugegeben und die Reaktionsmischung unter Stickstoffatmosphäre auf 60°C erwärmt. Nach einer Reaktionszeit von 4 h lässt man den Ansatz abkühlen und neutralisiert mit Salzsäure (19 wt-%) auf pH 6 - 7. Das Produkt wird mehrmals mit 80%-igem wässrigem Isopropanol und schließlich reinem Aceton gewaschen, getrocknet und gemahlen.

| Ansatz | Beispiel 1 | Beispiel 2 | Beispiel 3 |
|---|---|---|---|
| NaOH, g | 5,3 | 7,9 | 10,6 |
| N-(2-Chlorethyl)-N,N-diethylamin Hydrochlorid (65 wt-%), g | 15,9 | 23,8 | 31,8 |
| Roh-Ausbeute, g | 49,9 | 55,0 | 58,1 |
| Trockengehalt, % | 96,53 | 97,03 | 96,6 |
| Stickstoffgehalt, % bez. auf Trockenmasse | 1,55 | 2,16 | 2,61 |
| DS (N) | 0,21 | 0,31 | 0,4 |
| Ladungsdichte bei pH 11,5, mmol/g | 0 | 0,16 | 0,18 |
| Umsatz bez. auf Diethylaminoethylchlorid Hydrochlorid, % | 100 | 100 | 99 |
| Sulfatasche, % bez. auf Trockenmasse | 1,83 | 1,43 | 1,35 |
| Chloridgehalt, % bez. auf Trockenmasse | 2,35 | 3,63 | 5,1 |
| Viskosität (2 wt-% in Wasser), mPa*s | 51800 | 39830 | 33060 |
| Trübung (1 wt-% in Wasser), NTU | 41,7 | 18,4 | 13,3 |
| Transmission (1 wt-% in Wasser), % | 58,8 | 77 | 82,1 |
| Staudinger Index, mL/g | 1256 | 1411 | 1410 |

| Ansatz | Beispiel 4 | Beispiel 5 | Beispiel 6 |
|---|---|---|---|
| NaOH, g | 13,2 | 26,4 | 39,6 |
| N-(2-Chlorethyl)-N,N-diethylamin Hydrochlorid (65 wt-%), g | 39,7 | 79,4 | 119,2 |
| Roh-Ausbeute, g | 39,6 | 58,5 | 73,1 |
| Trockengehalt, % | 96,7 | 96,8 | 96,0 |

| Ansatz | Beispiel 4 | Beispiel 5 | Beispiel 6 |
|---|---|---|---|
| Stickstoffgehalt, % bez. auf Trockenmasse | 2,9 | 4,2 | 4,9 |
| DS (N) | 0,5 | 0,8 | 1,1 |
| Ladungsdichte bei pH 11,5, mmol/g | | 0,31 | 0,31 |
| Umsatz bez. auf Diethylaminoethylchlorid Hydrochlorid, % | 90 | 81 | 72 |
| Sulfatasche, % bez. auf Trockenmasse | 1,1 | 0,7 | 0,6 |
| Chloridgehalt, % bez. auf Trockenmasse | 5,4 | 9,0 | 11,8 |
| Viskosität (2 wt-% in Wasser), mPa*s | 27660 | 15350 | 12750 |
| Trübung (1 wt-% in Wasser), NTU | 10,7 | 5,6 | 4,5 |
| Transmission (1 wt-% in Wasser), % | 86,0 | 92,1 | 93,2 |
| Staudinger Index, mL/g | | 1177 | 1198 |

**Vergleichsbeispiele 1-5: Herstellung von Diethylaminoethylguar in einer Wasser in Öl Emulsion nach DE 2840011**

Guar (61 g, Trockengehalt 92,18 %, Staudinger Index: 1392 mL/g) wird in einer Mischung aus Octan (200 g) und Brij 92 (20 g) in einer 500 mL Glas-Vierhalsplanschliff-Rundboden Rührapparatur mit Impeller-Rührer suspendiert.
Die angegebene Menge N-(2-Chlorethyl)-N,N-diethylamin Hydrochlorid wird als wässrige Lösung (65 wt-%) zugegeben. Anschließend wird die angegebene Menge Natriumhydroxid als 50 %ige wässrige Lösung innerhalb von 45 Minuten zugegeben. Die Reaktionsmischung wird unter Stickstoffatmosphäre auf 70°C erwärmt. Nach einer Reaktionszeit von 4 h lässt man den Ansatz abkühlen und neutralisiert mit konzentrierter Salzsäure auf pH 4,5. Das Produkt wird mehrmals mit 80%-igem wässrigem Isopropanol und schließlich reinem Aceton gewaschen, getrocknet und gemahlen.

| Ansatz | Vergleich 1 | Vergleich 2 | Vergleich 3 |
|---|---|---|---|
| NaOH-Lösung (50 wt-%), g | 17,7 | 29,01 | 46,41 |
| N-(2-Chlorethyl)-N,N-diethylamin Hydrochlorid (65 wt-%), g | 26,7 | 46,3 | 74,1 |
| Roh-Ausbeute, g | 30,3 | 37,3 | 47,8 |
| Trockengehalt, % | 95,6 | 96,39 | 95,46 |
| Stickstoffgehalt, % bez. auf Trockenmasse | 1,4 | 2,93 | 3,77 |
| DS (N) | 0,18 | 0,47 | 0,74 |
| Ladungsdichte bei pH 11,5, mmol/g | 0 | 0,21 | 0,33 |
| Umsatz bez. auf Diethylaminoethylchlorid Hydrochlorid, % | 62 | 95 | 92 |
| Sulfatasche, % bez. auf Trockenmasse | 5,5 | 3,39 | 8,02 |
| Chloridgehalt, % bez. auf Trockenmasse | 2,5 | 6,55 | 11,6 |
| Viskosität (2 wt-% in Wasser), mPa*s | 13790 | 338 | 33,6 |
| Trübung (1 wt-% in Wasser), NTU | 81,1 | 8,3 | 6,5 |
| Transmission (1 wt-% in Wasser), % | 70,9 | 92,1 | 92,9 |
| Staudinger Index, mL/g | 776 | 403 | 256 |

| Ansatz | Vergleich 4 | Vergleich 5 |
|---|---|---|
| NaOH-Lösung (50 wt-%), g | 58,01 | 87,02 |
| N-(2-Chlorethyl)-N,N-diethylamin Hydrochlorid (65 wt-%), g | 93,0 | 139,0 |
| Roh-Ausbeute, g | 60,5 | 61,0 |
| Trockengehalt, % | 90,4 | 92,5 |

| Ansatz | Vergleich 4 | Vergleich 5 |
|---|---|---|
| Stickstoffgehalt, % bez. auf Trockenmasse | 4,18 | 5,03 |
| DS (N) | 0,88 | 1,12 |
| Ladungsdichte bei pH 11,5, mmol/g | 0,38 | 0,42 |
| Umsatz bez. auf Diethylaminoethylchlorid Hydrochlorid, % | 88 | 75 |
| Sulfatasche, % bez. auf Trockenmasse | 6,9 | 1,34 |
| Chloridgehalt, % bez. auf Trockenmasse | 13,3 | 12,1 |
| Viskosität (2 wt-% in 1 %iger Essigsäure), mPa*s | 27,6 | 31,2 |
| Trübung (1 wt-% in 1 %iger Essigsäure), NTU | 12,1 | 5,6 |
| Transmission (1 wt-% in 1 %iger Essigsäure), % | 88,5 | 95,7 |
| Staudinger Index, mL/g | 245 | 236 |

**Beispiele 7-9: Herstellung von Diisopropylaminoethylguar in einem Wasser-Dimethoxyethan-Slurry**

Guar (53 g, Trockengehalt 92,18 %, Staudinger Index: 1392 mL/g) wird in einer Mischung aus Dimethoxyethan (324 g) und Wasser (94 g) in einer 1 L Glas-Vierhalsplanschliff-Rundboden Rührapparatur mit Impeller-Rührer suspendiert.

Die angegebene Menge Natriumhydroxid wird als Feststoff zugegeben und nach 1 h bei Raumtemperatur wird die angegebene Menge N-(2-Chlorethyl)-N,N-diisopropylamin Hydrochlorid als wässrige Lösung (65 wt-%) zugegeben und die Reaktionsmischung unter Stickstoffatmosphäre auf 60°C erwärmt. Nach einer Reaktionszeit von 4 h lässt man den Ansatz abkühlen und neutralisiert mit Salzsäure (19 wt-%) auf pH 6 - 7. Das Produkt wird mehrmals mit 80%-igem wässrigem Isopropanol und schließlich reinem Aceton gewaschen, getrocknet und gemahlen.

| Ansatz | Beispiel 7 | Beispiel 8 | Beispiel 9 |
|---|---|---|---|
| NaOH, g | 13,2 | 26,4 | 39,6 |
| N-(2-Chlorethyl)-N,N-diisopropylamin Hydrochlorid (65 wt-%), g | 46,2 | 92,4 | 138,6 |
| Roh-Ausbeute, g | 68,1 | 87,5 | 77,7 |
| Trockengehalt, % | 96,46 | 97,18 | 96,80 |
| Stickstoffgehalt, % bez. auf Trockenmasse | 1,97 | 3,44 | 4,14 |
| DS (N) | 0,30 | 0,60 | 0,93 |
| Umsatz bez. auf Diisopropylaminoethylchlorid Hydrochlorid, % | 68 | 88 | 77 |
| Sulfatasche, % bez. auf Trockenmasse | 2,27 | 1,19 | 0,40 |
| Chloridgehalt, % bez. auf Trockenmasse | 4,87 | 1,19 | 10,2 |

(fortgesetzt)

| Ansatz | Beispiel 7 | Beispiel 8 | Beispiel 9 |
|---|---|---|---|
| Viskosität (2 wt-% in Wasser), mPa*s | 28990 | 15640 | 10320 |
| Trübung (1 wt-% in Wasser), NTU | 6,8 | 3,2 | 6,0 |
| Transmission (1 wt-% in Wasser), % | 93,3 | 98,6 | 94,5 |
| Staudinger Index, mL/g | 1419 | 1252 | 963 |

**Beispiele 10-12: Herstellung von Dimethylaminoethylguar in einem Wasser-Dimethoxyethan-Slurry**

Guar (88 g, Trockengehalt 92,18 %, Staudinger Index: 1392 mL/g) wird in einer Mischung aus Dimethoxyethan (540 g) und Wasser (164 g) in einer 1 L Glas-Vierhalsplanschliff-Rundboden Rührapparatur mit Impeller-Rührer suspendiert.

Die angegebene Menge Natriumhydroxid wird als Feststoff zugegeben und nach 1 h bei Raumtemperatur wird die angegebene Menge N-(2-Chlorethyl)-N,N-dimethylamin Hydrochlorid als wässrige Lösung (65 wt-%) zugegeben und die Reaktionsmischung unter Stickstoffatmosphäre auf 60°C erwärmt. Nach einer Reaktionszeit von 4 h lässt man den Ansatz abkühlen und neutralisiert mit Salzsäure (19 wt-%) auf pH 6 - 7. Das Produkt wird mehrmals mit 80%-igem wässrigem Isopropanol und schließlich reinem Aceton gewaschen, getrocknet und gemahlen.

| Ansatz | Beispiel 10 | Beispiel 11 | Beispiel 12 |
|---|---|---|---|
| NaOH, g | 22 | 44 | 66 |
| N-(2-Chlorethyl)-N,N-dimethylamin Hydrochlorid (65 wt-%), g | 55,4 | 110,8 | 166,2 |
| Roh-Ausbeute, g | 87,0 | 99,0 | 94,1 |
| Trockengehalt, % | 96,79 | 96,48 | 96,66 |
| Stickstoffgehalt, % bez. auf Trockenmasse | 2,2 | 3,38 | 3,99 |
| DS (N) | 0,3 | 0,5 | 0,66 |
| Umsatz bez. auf Dimethylaminoethylchlorid Hydrochlorid, % | 61 | 50 | 44 |
| Salfatasche, % bez. auf Trockenmasse | 1,52 | 1,39 | 1,06 |
| Chloridgehalt, % bez. auf Trockenmasse | 4,40 | 4,63 | 9,44 |
| Viskosität (2 wt-% in Wasser), mPa*s | 32630 | 23200 | 15840 |
| Trübung (1 wt-% in Wasser), NTU | 13,6 | 7,6 | 4,5 |
| Transmission (1 wt-% in Wasser), % | 83,5 | 91,7 | 95,7 |
| Staudinger Index, mL/g | 1383 | 1326 | 1209 |

**Vergleichsbeispiele 6-8: Herstellung von Diethylaminoethylguar in einem Wasser-Isopropanol-Slurry**

Guar (53 g, Trockengehalt 92,18 %, Staudinger Index: 1392 mL/g) wird in einer Mischung aus Isopropanol und Wasser entsprechend der unten aufgeführten Mengenangaben in einer 1 L Glas-Vierhalsplanschliff-Rundboden Rührapparatur mit Impeller-Rührer suspendiert.

Es werden 26,4 g Natriumhydroxid wird als Feststoff zugegeben und nach 1 h Rühren bei Raumtemperatur wird 79,4 g N-(2-Chlorethyl)-N,N-diethylamin Hydrochlorid-Lösung (65 wt-%) zugegeben und die Reaktionsmischung unter Stickstoffatmosphäre auf 60°C erwärmt. Nach einer Reaktionszeit von 4 h lässt man den Ansatz abkühlen und neutralisiert mit Salzsäure (19 wt-%) auf pH 6 - 7. Das Produkt wird mehrmals mit 80%-igem wässrigem Isopropanol und schließlich reinem Aceton gewaschen, getrocknet und gemahlen.

| Ansatz | Vergleich 6 | Vergleich 7 | Vergleich 8 |
|---|---|---|---|
| Isopropanol | 648 | 686 | 724 |
| Wasser | 83 | 44 | 6 |

(fortgesetzt)

| Ansatz | Vergleich 6 | Vergleich 7 | Vergleich 8 |
|---|---|---|---|
| Roh-Ausbeute, g | 37,5 | 37,1 | 30,1 |
| Trockengehalt, % | 95,9 | 95,6 | 96,1 |
| Stickstoffgehalt, % bez. auf Trockenmasse | 0,75 | 0,56 | 0,58 |
| DS (N) | 0,09 | 0,07 | 0,07 |
| Umsatz bez. auf Diethylaminoethylchlorid Hydrochlorid, % | 9,4 | 6,9 | 7,2 |
| Sulfatasche, % bez. auf Trockenmasse | 2,17 | 2,24 | 3,32 |
| Chloridgehalt, % bez. auf Trockenmasse | 1,57 | 0,99 | 1,42 |
| Viskosität (2 wt-% in Wasser), mPa*s | 50250 | 37190 | 53810 |
| Trübung (1 wt-% in Wasser), NTU | 124 | 180 | 119 |
| Transmission (1 wt-% in Wasser), % | 42,9 | 32,4 | 36,8 |

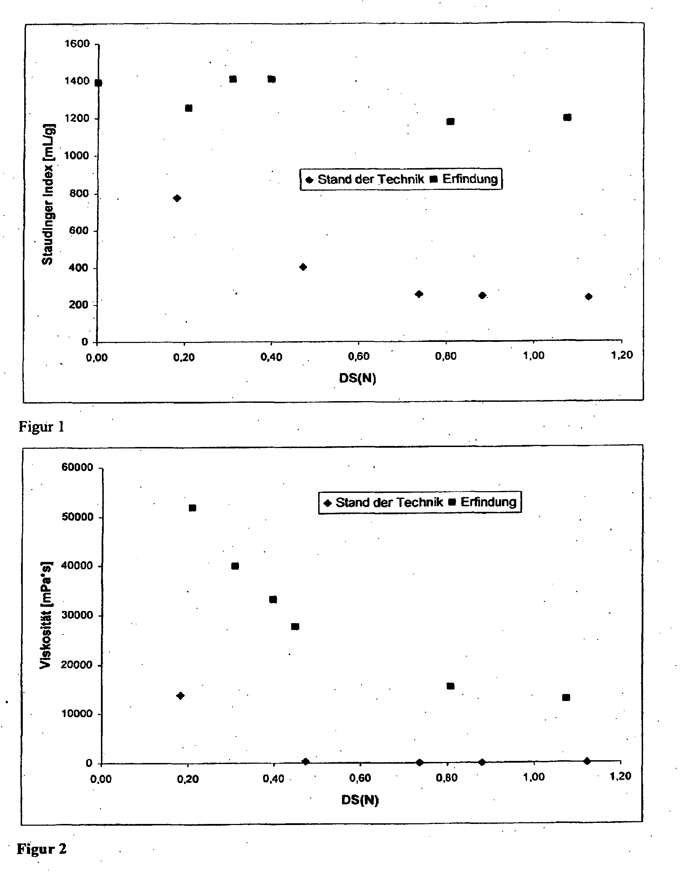

Figur 1 zeigt einen Vergleich der Staudinger Indices in 0,1 M NaCl von Diethylaminoethylguar, hergestellt nach dem erfindungsgemäßen Slurryverfahren (Beispiele 1 bis 6, Quadrate) und dem bekannten Emulsionsverfahren nach DE28 40 011 (Vergleichsbeispiele 7 bis 11, Rauten).

Die nach dem Emulsionsverfahren hergestellten Diethylaminoethylguar zeigen eine deutliche Abnahme im Staudinger Index mit zunehmendem Substitutionsgrad. Im Gegensatz dazu lassen sich nach dem neuen Verfahren Aminoalkylguar-Derivate herstellen, die auch bei hohem Substitutionsgrad hohe Staudinger Indices und damit hohe Molekulargewichte aufweisen. Wie in der Abbildung deutlich wird, nehmen die Staudinger Indices mit zunehmendem Substitutionsgrad gar nicht oder nur gering ab.

In Figur 2 werden die Viskositäten in Wasser (2 wt-%) von erfindungsgemäß hergestellten Diethylaminoethylguar (Beispiele 1 bis 6, Quadrate) in Abhängigkeit zum Substitutionsgrad DS(N) (durchschnittliche Zahl Aminogruppen pro Anhydrozucker-Einheit) dargestellt. Zum Vergleich werden solche Guar-Derivate mit aufgeführt, die nach dem oben genannten Emulsionsverfahren hergestellt wurden (Vergleichsbeispiele 7 bis 11, Rauten).

**Patentansprüche**

1. Verfahren zur Herstellung aminoalkylgruppenhaltiger Guar-Derivate, wobei man

   a) Guar oder ein Guar-Derivat in einem Gemisch (Slurry) aus einem wassermischbaren aprotischen Lösungsmittel und Wasser dispergiert und

   b) das Guar oder Guar-Derivat mit einer Base alkalisiert und

   c) anschließend eine Veretherung mit einem aminogruppenhaltigen Reagenz bei gegebenenfalls erhöhter Temperatur durchführt und dann

   d) gegebenenfalls neutralisiert und

   e) das Reaktionsprodukt gegebenenfalls abfiltriert, gegebenenfalls wäscht, gegebenenfalls trocknet und gegebenenfalls mahlt, **dadurch gekennzeichnet, dass** das aminogruppenhaltige Reagenz eine Verbindungen der allgemeinen Formel

   $$X\text{-}(CH_2)_n\text{-}NR_1R_2 \text{ ist}$$

   wobei

   X eine Fluchtgruppe ausgewählt aus Chlor, Brom, Iod oder ein Sulfonsäurerest $R'SO_3$ ist, wobei $R'$ ein aromatischer oder aliphatischer Rest ist,
   n eine ganze Zahl ist, die mindestens 2 sein muss,

   die Reste $R_1$ und $R_2$ unabhängig voneinander aliphatische oder verzweigte oder zyklische, gegebenenfalls mit Heteroatomen substituierte Alkyl- oder Aryl-Substituenten mit 1-24 C-Atomen oder H bedeuten oder zwei Reste $R_1$ und $R_2$ gemeinsam mit dem Stickstoffatom einen Ring bilden können.

**2.** Verfahren gemäß Anspruch 1, **dadurch gekennzeichnet, dass** das wassermischbare aprotische Lösungsmittel ausgewählt ist aus der Gruppe bestehend aus Aceton, Methylethylketon, Dimethoxyethan.

**3.** Verfahren gemäß Anspruch 1 oder 2, **dadurch gekennzeichnet, dass** das aminogruppenhaltige Reagenz ausgewählt ist aus der Gruppe bestehend aus N-(2-Chlorethyl)-N,N-diisopropyl-amin, N-(2-Chlorethyl)-N,N-diethylamin oder N-(2-Chlorethyl)-N,N-dimethylamin oder den Hydrochloriden dieser Amine.

**4.** Verfahren gemäß einem der Ansprüche 1 bis 3, **dadurch gekennzeichnet, dass** das aminogruppenhaltige Reagenz in einer Menge von 0,1 - 3 mol pro mol Anhydrozucker des Guars oder Guar-Derivats eingesetzt wird.

**5.** Verfahren gemäß einem der Ansprüche 1 bis 4, **dadurch gekennzeichnet, dass** als Ausgangsmaterial ein Guarether eingesetzt wird.

**6.** Aminoalkylgruppenhaltige Guar-Derivate, **dadurch gekennzeichnet, dass** ein Teil der Hydroxylgruppen in Dialkylaminoalkylethergruppen der allgemeinen Formel

$$O\text{-}(CH_2)_n\text{-}NR_1R_2$$

wobei

n eine ganze Zahl ist, die mindestens 2 sein muss,

die Reste $R_1$ und $R_2$ unabhängig voneinander aliphatische oder verzweigte oder zyklische, gegebenenfalls mit Heteroatomen substituierte Alkyl- oder Aryl-Substituenten mit 1-24 C-Atomen oder H bedeuten oder zwei Reste $R_1$ und $R_2$ gemeinsam mit dem Stickstoffatom einen Ring bilden können,
überführt wurden und diese aminoalkylgruppenhaltigen Guar-Derivate einen Staudinger-Index > 900 mL/g, gemessen in einem Kapillarviskosimeter nach Ubbelohde bei 20°C in 0,1 M NaCl-Lösung, und einen DS(N) > 0,3, gemessen nach der Kjeldahl-Methode, aufweisen.

**7.** Aminoalkylgruppenhaltige Guar-Derivate gemäß Anspruch 6, **dadurch gekennzeichnet, dass** die Aminoalkylgruppen zumindestens teilweise in quaternisierter Form vorliegen.

**8.** Verwendung von aminoalkylgruppenhaltigen Guar-Derivaten gemäß einem der Ansprüche 6 bis 7 zur Herstellung von Haarkonditionierern.

**9.** Verwendung von aminoalkylgruppenhaltigen Guar-Derivaten gemäß einem der Ansprüche 6 bis 7 zur Herstellung von Flockungshilfsmitteln.

**Claims**

**1.** A method for producing aminoalkyl group-containing guar derivatives comprising

a) dispersing guar or guar derivatives in a mixture (slurry) of a water-miscible aprotic solvent and water and
b) alkalizing the guar or guar derivative with a base and
c) subsequently etherifying said mixture with an amino group-containing reagent, optionally at an elevated temperature, and then
d) optionally neutralizing said mixture
e) optionally filtering, optionally washing, optionally drying and optionally grinding the reaction product, **characterized in that** the amino group-containing reagent is a compound of the general formula

$$X\text{-}(CH_2)_n\text{-}NRiR_2$$

wherein

X is a leaving group selected from chlorine, bromine, iodine or a sulphonic acid radical $R'SO_3$, wherein R' is an aromatic or aliphatic radical
n is an integer which must be at least 2,

the radicals $R_1$ and $R_2$ are independently of one another aliphatic or branched or cyclic alkyl or aryl substituents that may be optionally substituted by heteroatoms, having 1-24 carbon atoms, or H, or the two radicals $R_1$ and $R_2$ together with the nitrogen atom may form a ring.

2. The method according to claim 1, **characterized in that** the water-miscible aprotic solvent is selected from the group consisting of acetone, methyl ethyl ketone and dimethoxyethane.

3. The method according to claim 1 or 2, **characterized in that** the amino group-containing reagent is selected from the group consisting of N-(2-chloroethyl)-N,N-diisopropylamine, N-(2-chloroethyl)-N,N-diethylamine or N-(2-chloroethyl)-N,N-dimethylamine or the hydrochlorides of these amines.

4. The method according to any of claims 1 to 3, **characterized in that** the amino group-containing reagent is used in an amount of 0.1 to 3 mole per mole of anhydrous sugar of the guar or the guar derivative.

5. The method according to any of claims 1 to 4, **characterized in that** guar ether is used as starting material.

6. Aminoalkyl group-containing guar derivatives, **characterized in that** a part of the hydroxyl groups is converted into dialkylaminoalkyl ether of the general formula

$$O\text{-}(CH_2)_n\text{-}NR_1R_2$$

wherein

n is an integer which must be at least 2,
the radicals $R_1$ and $R_2$ are independently of one another aliphatic or branched or cyclic alkyl or aryl substituents that may be optionally substituted by heteroatoms, having 1-24 carbon atoms, or H, or the two radicals $R_1$ and $R_2$ together with the nitrogen atom may form a ring,
and these amino group-containing guar derivatives have a Staudinger index >900 ml/g, measured in a capillary viscosimeter according to Ubbelohde at 20°C in 0.1 M NaCl solution and a DS(N) >0.3, measured according to the Kjeldahl method.

7. Amino group-containing guar derivatives according to claim 6, **characterized in that** the aminoalkyl groups are present at least in part in quaternized form.

8. Use of the amino group-containing guar derivatives according to any of claims 6 to 7 for the production of hair conditioners.

9. Use of the amino group-containing guar derivatives according to any of claims 6 to 7 for the production of flocculation aids.

## Revendications

1. Procédé de préparation de dérivés de guar contenant des groupes aminoalkyle, dans lequel

a) on disperse du guar ou un dérivé de guar dans un mélange (suspension épaisse) constitué d'un solvant aprotique miscible à l'eau et d'eau et
b) on alcalinise le guar ou le dérivé de guar par une base et
c) on réalise ensuite une éthérification avec un réactif contenant des groupes amino à une température éventuellement augmentée, puis
d) on la neutralise éventuellement et
e) on filtre éventuellement le produit de réaction, le lave éventuellement, le sèche éventuellement et le moud éventuellement, **caractérisé en ce que** le réactif contenant des groupes amino est un composé de la formule générale

$$X\text{-}(CH_2)_n\text{-}NR_1R_2$$

dans laquelle

X est un groupe partant choisi parmi le chlore, le brome, l'iode ou un reste d'acide sulfonique $R'SO_3$, où R' est un reste aromatique ou aliphatique,

n est un nombre entier qui doit au moins être 2,

les restes $R_1$ et $R_2$, indépendamment l'un de l'autre, représentent des substituants alkyle ou aryle aliphatiques ou ramifiés ou cycliques, éventuellement substitués par des hétéroatomes, avec de 1 à 24 atomes de C ou H ou deux restes $R_1$ et $R_2$ peuvent former un cycle conjointement avec l'atome d'azote.

2. Procédé selon la revendication 1, **caractérisé en ce que** le solvant aprotique miscible à l'eau est choisi dans le groupe constitué de l'acétone, de la méthyléthylcétone, du diméthoxyéthane.

3. Procédé selon la revendication 1 ou 2, **caractérisé en ce que** le réactif contenant des groupes amino est choisi dans le groupe constitué de la N-(2-chloroéthyle)-N,N-diisopropylamine, de la N-(2-chloroéthyle)-N,N-diéthylamine ou de la N-(2-chloroéthyle-N,N-diméthylamine ou des chlorhydrates de ces amines.

4. Procédé selon l'une des revendications 1 à 3, **caractérisé en ce que** le réactif contentant des groupes amino est utilisé dans une quantité allant de 0,1 à 3 moles par mole de sucre anhydre du guar ou du dérivé de guar.

5. Procédé selon l'une des revendications 1 à 4, **caractérisé en ce qu'**un éther de guar est utilisé comme matière de base.

6. Dérivés de guar contenant des groupes aminoalkyle, **caractérisés en ce qu'**une partie des groupes hydroxyle a été convertie en groupes d'éther de dialkylaminoalkyle de la formule générale
$O(CH_2)_n-NR_1R_2$

n est un nombre entier qui doit au moins être 2,

les restes $R_1$ et $R_2$, indépendamment l'un de l'autre, représentent des substituants alkyle ou aryle aliphatiques ou ramifiés ou cycliques, éventuellement substitués par des hétéroatomes, avec de 1 à 24 atomes de C ou H ou deux restes $R_1$ et $R_2$ peuvent former un cycle conjointement avec l'atome d'azote, et ces dérivés de guar contenant des groupes aminoalkyle présentent un indice de Staudinger > 900 mL/g mesuré à 20°C dans un viscosimètre à capillaire selon Ubbelohde dans une solution de NaCl 0,1 M, et un DS(N) > 0,3, mesuré selon la méthode de Kjeldahl.

7. Dérivés de guar contenant des groupes aminoalkyle selon la revendication 6, **caractérisés en ce que** les groupes aminoalkyle sont présents au moins partiellement sous une forme quaternisée.

8. Utilisation de dérivés de guar contenant des groupes aminoalkyle selon l'une des revendications 6 à 7 pour la préparation de revitalisants capillaires.

9. Utilisation de dérivés de guar contenant des groupes aminoalkyle selon l'une des revendications 6 à 7 pour la préparation d'agents floculants.

Figur 1

Figur 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0175113 A2
- DE 2840011
- US 4276414 A
- US 3498912 A
- US 20010051143 A1
- EP 0310787 A

### In der Beschreibung aufgeführte Nicht-Patentliteratur

- Designs of Solid - Solid Mixers, 3.3 Paddle Mixers. Ullmann's Encyclopedia of Industrial Chemistry. Wiley-VCH Verlag GmbH & Co. KgaA, 15. Juni 2000